# EUROPEAN PATENT APPLICATION

(11) **EP 2 064 986 A1**
(43) Date of publication of application: **03.06.2009**
(21) Application number: 07807222.0
(22) Date of filing: 13.09.2007
(51) Int. Cl.: A61B 1/04, A61B 1/00, A61B 1/06, G02B 23/24, H04N 7/18

(54) **ENDOSCOPE SYSTEM**

(30) Priority: 21.09.2006 JP 2006256296
(71) Applicant: Olympus Medical Systems Corp., Tokyo 151-0072 (JP)
(72) Inventor: URAKAWA, Tsutomu, Tokyo 151-0072 (JP); HONDA, Takemitsu, Tokyo 151-0072 (JP)
(74) Representative: von Hellfeld, Axel
(86) International application number: PCT/JP2007/067815
(87) International publication number: WO 2008/035608

(57) **Abstract**

An endoscope system of the invention includes an elongated insertion portion; an image pickup portion provided with an objective optical system having an optical axis diagonal to a longitudinal axis direction of the insertion portion and that picks up an image of a subject present in a field of view of the objective optical system and can rotate around the longitudinal axis of the insertion portion; a rotation angle detection portion that detects a rotation angle of the image pickup portion; an image pickup control portion that can have images of a plurality of subjects picked up while the image pickup portion makes one rotation by controlling the image pickup portion on the basis of the rotation angle detected by the rotation angle detection portion; and a signal processing portion that carries out signal processing for having each image of the plurality of subjects displayed on one display portion.

## Description

### Technical Field

The present invention relates to an endoscope system and particularly to an endoscope system that can display an image of a plurality of subjects on one display portion.

### Background Art

An endoscope system configured by having an endoscope and the like has been widely used in the medical field and the industrial field and the like. Also, the endoscope system is used when a portion and a tissue and the like in a body cavity of a living body, which is a lesion, is observed in the medical field. An image pickup apparatus pursuant to the above-mentioned endoscope system is proposed in Japanese Patent Application Laid-Open Publication No. 11-089793, for example.

The side-view type image pickup apparatus in Japanese Patent Application Laid-Open Publication No. 11-089793 is configured by having a hand-piece provided with an image pickup system for forming and picking up an image of a subject incoming from a translucent window on a side face of a distal end portion and a head portion rotatable with respect to an optical axis of the image pickup system and a signal processor that processes an image pickup signal outputted from the image pickup system and outputs the signal to an image display device. And the side-view type image pickup apparatus in Japanese Patent Application Laid-Open Publication No. 11-089793 can output an image of a desired subject present in a radial direction with respect to the optical axis of the image pickup system by having the above-mentioned configuration to the image display device.

However, since the side-view type image pickup apparatus in Japanese Patent Application Laid-Open Publication No. 11-089793 has only one image of the subject displayed in a screen of the image display device, there is a problem that a range capable of observation at one time depends on a view angle of the transmission window as an objective optical system.

The present invention was made in view of the above points and has an object to provide an endoscope system that can enlarge a range capable of observation at one time than before.

### Disclosure of Invention

### Means for Solving the Problem

The endoscope system of the present invention includes an elongated insertion portion; an image pickup portion provided with an objective optical system having an optical axis diagonal to a longitudinal axis direction of the insertion portion and that picks up an image of a subject present in a field of view of the objective optical system and can rotate around the longitudinal axis of the insertion portion; a rotation angle detection portion that detects a rotation angle of the image pickup portion; an image pickup control portion that can have images of a plurality of subjects picked up while the image pickup portion makes one rotation by controlling the image pickup portion on the basis of the rotation angle detected by the rotation angle detection portion; and a signal processing portion that carries out signal processing for having each image of the plurality of subjects displayed on one display portion.

### Brief Description of the Drawings

Fig. 1 is a diagram illustrating an example of configuration of an essential part of an endoscope system according to the present embodiment;
Fig. 2 is a diagram illustrating an example of configuration of a distal end portion provided at an insertion portion of the endoscope system in Fig. 1;
Fig. 3 is a diagram illustrating an example of a display state of an image according to an image of a subject picked up with rotation of the insertion portion in Fig. 1;
Fig. 4 is a diagram illustrating an example of a display state when one image is selected from each image displayed in Fig. 3;
Fig. 5 is a diagram illustrating an example of a display state of a synthetic image, which is an image formed by synthesizing each image displayed in Fig. 3;
Fig. 6 is a diagram illustrating an example different from Fig. 2 of the distal end portion provided at the insertion portion of the endoscope system in Fig. 1;
Fig. 7 is a diagram illustrating an example different from Fig. 2 and Fig. 6 of the distal end portion provided at the insertion portion of the endoscope system in Fig. 1;
Fig. 8 is a diagram illustrating configuration of each connector portion in Fig. 7;
Fig. 9 is a diagram illustrating configuration of one connector portion in each connector portion in Fig. 8, when seen from an arrow S direction; and
Fig. 10 is a diagram illustrating a state when each connector portion in Fig. 8 is connected.

### Best Mode for Carrying Out the Invention

An embodiment of the present invention will be described below referring to the attached drawings.

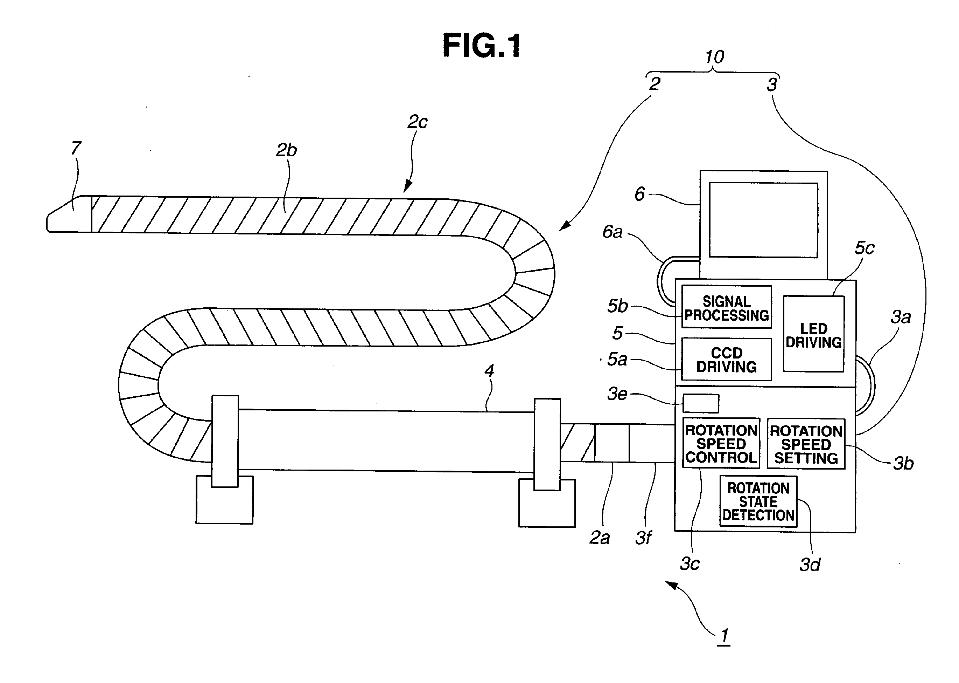

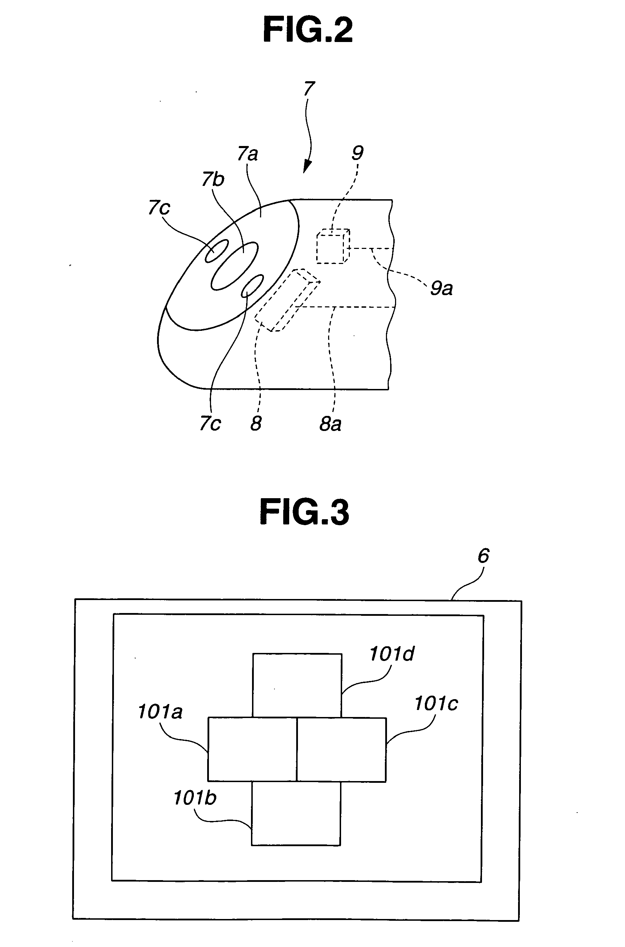

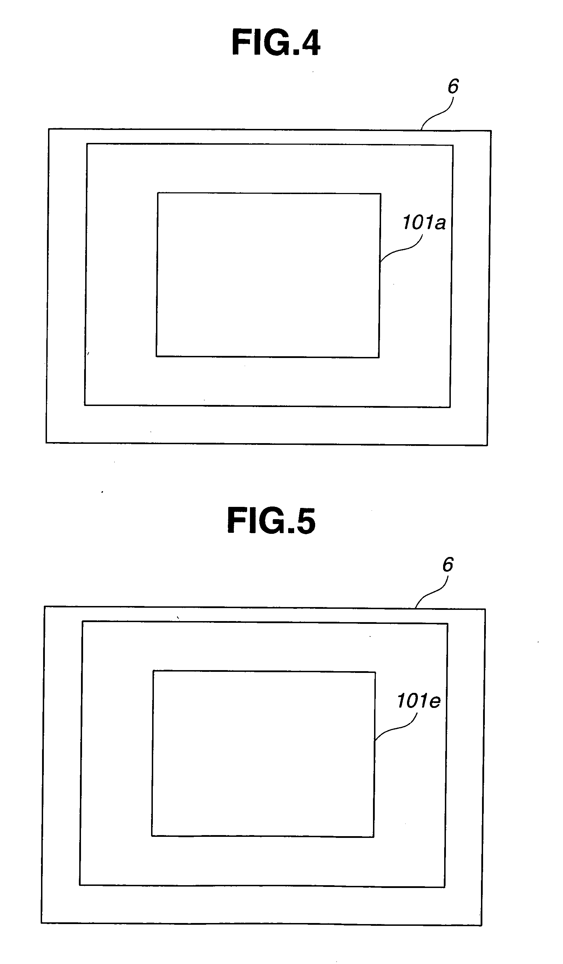

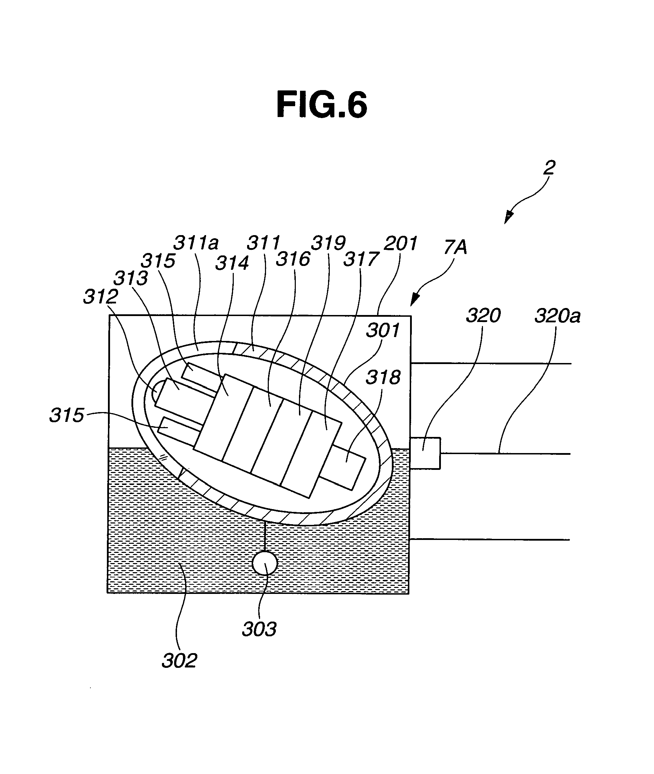

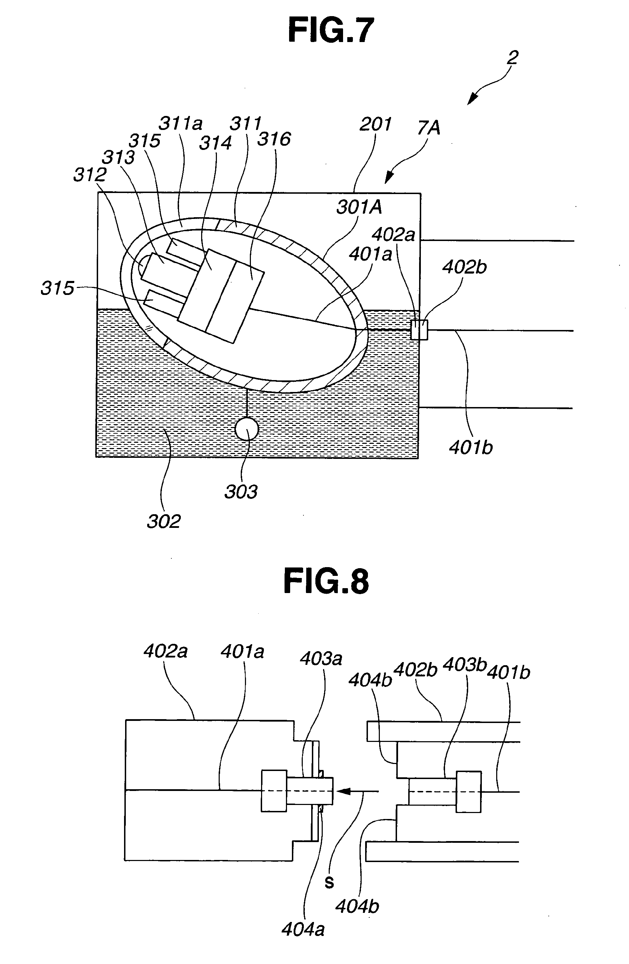

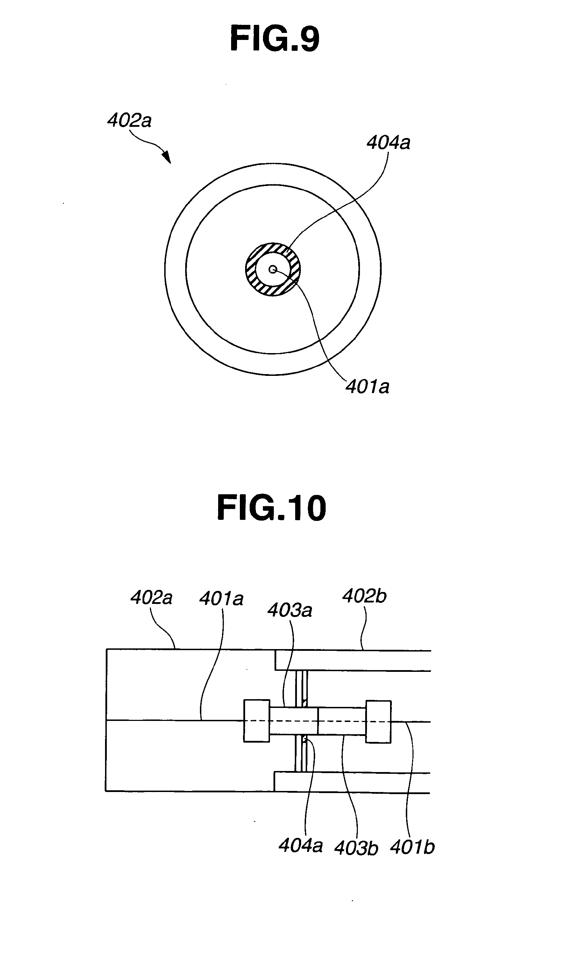

Figs. 1 to 10 relate to the embodiment of the present invention. Fig. 1 is a diagram illustrating an example of configuration of an essential part of the endoscope system according to the present embodiment. Fig. 2 is a diagram illustrating an example of configuration of a distal end portion provided at an insertion portion of the endoscope system in Fig. 1. Fig. 3 is a diagram illustrating an example of a display state of an image according to an image of a subject picked up with rotation of the insertion portion in Fig. 1. Fig. 4 is a diagram illustrating an example of a display state when one image is selected from each image displayed in Fig. 3. Fig. 5 is a diagram illustrating an example of a display state of a synthetic image, which is an image formed by synthesizing each image displayed in Fig. 3. Fig. 6 is a diagram illustrating an example different from Fig. 2 of the distal end portion provided at the insertion portion of the endoscope system in Fig. 1. Fig. 7 is a diagram illustrating an example different from Fig. 2 and Fig. 6 of the distal end portion provided at the insertion portion of the endoscope system in Fig. 1. Fig. 8 is a diagram illustrating configuration of each connector portion in Fig. 7. Fig. 9 is a diagram illustrating configuration of one connector portion in each connector portion in Fig. 8, when seen from an arrow S direction. Fig. 10 is a diagram illustrating a state when each connector portion in Fig. 8 is connected.

The endoscope system 1 has as essential parts, as shown in Fig. 1, as configuration that can be inserted into a body cavity, an insertion portion 2 that is elongated and flexible, for example, a rotating device 3 to which the insertion portion 2 is detachably connected for rotating the connected insertion portion 2 in a predetermined direction around a longitudinal axis, a protective tube 4 for holding the insertion portion 2 rotatably by means of insertion thereof, a video processor 5 connected to the rotating device 3 through a signal cable 3a, and a monitor 6 as a display portion connected to the video processor 5 through a cable 6a and for displaying an image of a subject according to a video signal outputted from the video processor 5. And an endoscope 10 in the endoscope system 1 of the present embodiment is configured by having the insertion portion 2 and the rotating device 3.

In addition, the insertion portion 2 has a connector portion 2a configured detachable with respect to an insertion portion holding portion 3f provided at the rotating device 3 on a proximal end side.

Moreover, the insertion portion 2 has a distal end portion 7 rotating with rotation of the insertion portion 2 as shown in Fig. 2 on a distal end side. Specifically, the distal end portion 7 includes a distal end face 7a formed diagonally to an insertion axis direction of the insertion portion 2, an objective optical system 7b provided substantially at the center of the distal end face 7a for forming an image of a subject present in a field of view, and LED 7c provided each so as to sandwich the objective optical system 7b and emitting illumination light to the subject in the field of view of the objective optical system 7b.

The objective optical system 7b as a part of an image pickup portion is configured by a lens and the like having a predetermined view angle of approximately 100 to 140 degrees, for example, and is arranged so as to have an optical axis diagonal to the insertion axis (longitudinal axis) direction of the insertion portion 2.

Also, a CCD (charge coupled device) 8 is arranged at a position inside the distal end portion 7 and corresponding to an image forming position of the objective optical system 7b. Moreover, inside the distal end portion 7, a triaxial sensor 9 is arranged as a part of the rotation angle detection portion for detecting a position and an inclination from a horizontal state of the distal end portion 7 by detecting a magnetic field such as earth magnetism emitted outside the insertion portion 2 and for outputting information of the detected position and the inclination as a position information signal through a signal line 9a.

The CCD 8 as a part of the image pickup portion picks up an image of the subject formed by the objective optical system 7b, converts the image of the subject to an image pickup signal and outputs the signal. The image pickup signal outputted from the CCD 8 is inputted into the video processor 5 through a signal line 8a provided so as to be inserted through the insertion portion 2 and the signal cable 3a and the like.

The image pickup portion in the endoscope system 1 is configured having the above-mentioned objective optical system 7b and the CCD 8. The above-mentioned position information signal indicates the position and the inclination from the horizontal state of the distal end portion 7 and similarly indicates the position and the inclination from the horizontal state of the image pickup portion.

The video processor 5 has a CCD driving portion 5a as an image pickup control portion for controlling driving of the CCD 8, a signal processing portion 5b for carrying out signal processing to an image pickup signal outputted from the CCD 8 and outputting the signal as a video signal to the monitor 6, and an LED driving portion 5c for controlling light emission and driving of the LED 7c inside. With such configuration, an image of the subject picked up by the CCD 8 is outputted as an image pickup signal and converted by the signal processing portion 5b to a video signal and then, the image is displayed on the monitor 6.

The rotating device 3 is configured by a dial or a plurality of switches and the like, for example, and has a rotation speed setting portion 3b that can set a rotation speed of the insertion portion 2 (or the distal end portion 7 rotated with the insertion portion 2) to a value of a rotation speed desired by a user, a rotation speed control portion 3c configured by a motor and the like, for example, for controlling the rotation speed of the insertion portion 2 to the value set at the rotation speed setting portion 3b, and a rotation state detection portion 3d configured by a signal amplification portion and a decoder and the like, for example, for amplifying the position information signal outputted from the triaxial sensor 9 through the signal line 9a and for detecting the rotation angle of the distal end portion 7 on the basis of the position information signal. Also, the rotating device 3 is configured by having a rotation speed display portion 3e displaying a value of the rotation speed set at the rotation speed setting portion 3b and the insertion portion holding portion 3f configured detachably to the connector portion 2a of the insertion portion 2 on an outer surface thereof.

The rotation speed setting portion 3b outputs the value of the rotation speed of the insertion portion 2 set by the user not only to the rotation speed control portion 3c but also to each portion of the video processor 5 through the signal cable 3a. Thereby, each portion of the video processor 5 is operated according to the value of the rotation speed of the insertion portion 2 set by the user.

Specifically, the CCD driving portion 5a can drive the CCD 8 according to the value of the rotation speed of the insertion portion 2. The signal processing portion 5b carries out signal processing to the image pickup signal according to the value of the rotation speed of the insertion portion 2 and moreover, the portion can output a video signal according to the value of the rotation speed of the insertion portion 2 to the monitor 6. Also, the LED driving portion 5c can drive and have the LED 7c emit light on the basis of the driving timing of the CCD 8 set by the CCD driving portion 5a according to the value of the rotation speed of the insertion portion 2.

The rotation state detection portion 3d as a part of the rotation angle detection portion detects a rotation angle of the distal end portion 7 on the basis of the position information signal outputted from the triaxial sensor 9 through the signal line 9a and outputs the angle information, which is information relating to the rotation angle, to the CCD driving portion 5a of the video processor 5 through the signal cable 3a.

Thereby, the CCD driving portion 5a can drive the CCD 8 according to the value of the rotation speed set at the rotation speed setting portion 3b and also can have an image of a subject picked up as appropriate each time the rotation angle of the distal end portion 7 reaches a plurality of predetermined angles on the basis of the angle information outputted from the rotation state detection portion 3d.

The rotation angle detection portion in the endoscope system 1 is configured including the above-mentioned rotation state detection portion 3d and the triaxial sensor 9.

Also, in the insertion portion 2, on the outer circumferential face between the distal end portion 7 and the connector portion 2a, a sufficiently long thrust tube (or a guide tube) 2b having a spiral shaped portion 2c generating a thrust by a rotation operation of the rotation speed control portion 3c is formed.

An action of the endoscope system 1 will be described below.

First, a user powers on each portion of the endoscope system 1 and then, connects the connector portion 2a to the insertion portion holding portion 3f. Then, the user sets a value of a desired rotation speed at the rotation speed setting portion 3b and then, inserts the insertion portion 2 controlled to the desired rotation speed by the rotation speed control portion 3c into a body cavity.

The triaxial sensor 9 detects a position and an inclination from the horizontal state of the distal end portion 7 rotating with the insertion portion 2 on the basis of a magnetic field such as earth magnetism emitted outside the insertion portion 2 and outputs the information of the detected position and the inclination as a position information signal through the signal line 9a.

The rotation state detection portion 3d amplifies the position information signal outputted from the triaxial sensor 9 and detects the rotation angle of the distal end portion 7 on the basis of the position information signal and outputs the angle information, which is information relating to the rotation angle to the CCD driving portion 5a of the video processor 5 through the signal cable 3 a.

The CCD driving portion 5a determines how many frames of the image of the subject are to be picked up in one rotation of the insertion portion 2 by driving the CCD 8 according to the value of the rotation speed set at the rotation speed setting portion 3b. Specifically, for example, if the rotation speed of the insertion portion 2 is set at 10 rotations per second by the rotation speed setting portion 3b and if the image of the subject for four frames are to be picked up while the insertion portion 2 makes one rotation, the CCD driving portion 5a drives the CCD 8 so that the image of the subject for 1 frame per 25 ms can be picked up. In other words, for example, if the rotation speed of the insertion portion 2 is set at N rotations per second by the rotation speed setting portion 3b and if the image of the subject for M frames is to be picked up while the insertion portion 2 makes one rotation, the CCD driving portion 5a drives the CCD 8 so that the image of the subject for one frame per (1000/(M x N)) ms can be picked up.

Moreover, the CCD driving portion 5a has the image of the subject picked up as appropriate in a state where the rotation angle of the distal end portion 7 from the horizontal state reaches each of the rotation angles of 90 degrees, 180 degrees, 270 degrees, and 360 degrees, for example, on the basis of the angle information outputted from the rotation state detection portion 3d. As a result, the CCD 8 sequentially outputs the image of the subject in the state where the rotation angle from the horizontal state reaches each of the rotation angles of 90 degrees, 180 degrees, 270 degrees, and 360 degrees while the distal end portion 7 makes one rotation as an image pickup signal. The horizontal state in the present embodiment is set as a state where the distal end portion 7 is located in a state shown in Fig. 1. Also, in the following explanation, the insertion portion 2 rotates counterclockwise with respect to the insertion axis direction, for example, as a predetermined direction around the longitudinal axis. However, the insertion portion 2 of the present embodiment is not limited to one rotated counterclockwise but may be one rotating clockwise.

The signal processing portion 5b carries out the signal processing for having the image of the subject for four frames picked up while the distal end portion 7 makes one rotation, respectively, displayed as a still image in a screen of the monitor 6 to the image pickup signal outputted from the CCD 8 and outputs the image pickup signal after the signal processing is applied to the monitor 6 as a video signal.

Thereby, the monitor 6 displays the image of the subject arranged according to the rotation angle from the horizontal state of the distal end portion 7 and the rotation direction of the insertion portion 2 as a still image, respectively.

Specifically, as shown in Fig. 3, the image of the subject when the distal end portion 7 is rotated counterclockwise by 90 degrees with respect to the insertion axis direction of the insertion portion 2 is displayed as an observation image 101a, which is a still image arranged to the left in the screen of the monitor 6. Also, as shown in Fig. 3, the image of the subject when the distal end portion 7 is rotated counterclockwise by 180 degrees with respect to the insertion axis direction of the insertion portion 2 is displayed as an observation image 101b, which is a still image arranged to the lower part in the screen of the monitor 6. Moreover, as shown in Fig. 3, the image of the subject when the distal end portion 7 is rotated counterclockwise by 270 degrees with respect to the insertion axis direction of the insertion portion 2 is displayed as an observation image 101c, which is a still image arranged to the right in the screen of the monitor 6. And as shown in Fig. 3, the image of the subject when the distal end portion 7 is rotated counterclockwise by 360 degrees with respect to the insertion axis direction of the insertion portion 2 is displayed as an observation image 101c, which is a still image arranged to the upper part in the screen of the monitor 6. Each of the observation images 101a, 101b, 101c, and 101d shown in Fig. 3 is arranged at positions not overlapping with each other in the screen of the monitor 6 by means of the signal processing by the signal processing portion 5b, for example. Also, each of the observation images 101a, 101b, 101c, and 101d is not limited to those arranged at positions not overlapping with each other in the screen of the monitor 6 (as shown in Fig. 3) but may be those arranged at positions where some of all of the observation images overlap with each other, for example.

Each of the observation images in the screen of the monitor 6 is not limited to those displayed as a display state as shown in Fig. 3 but may be those displayed by being switched to another display state different from that shown in Fig. 3 by means of a display mode switch, not shown, capable of switching the display state of each of the observation images, for example.

Specifically, each of the observation images may be displayed in the screen of the monitor 6 by being switched to one of display modes of a display mode as in Fig. 3 where all the observation images 101a, 101b, 101c, and 101d are displayed at once, a display mode as in Fig. 4 where any one of the observation images 101a, 101 b, 101c, and 101d is displayed in an enlarged manner, and a display mode as in Fig. 5 where a synthetic image 101e, which is an image formed by synthesizing the observation images 101a, 101b, 101c, and 101d (such as a panorama image) by the signal processing on the basis of the operation of the display mode switch carried out at the signal processing portion 5b. Fig. 4 shows an example of a display state when the observation image 101a in the respective observation images is selected and displayed in an enlarged manner.

As mentioned above, the endoscope system 1 of the present invention has configuration in which an image of the subject present in each direction of up, down, right and left with respect to the insertion axis direction of the insertion portion 2 can be observed at once in the monitor 6. As a result, the endoscope system 1 of the present embodiment can expand a range capable of observation at once than before.

When the endoscope that can be inserted into a body cavity while the insertion portion is being rotated and is provided with the objective optical system having the optical axis diagonal to the insertion axis direction of the insertion portion is used, there might be a case where the user can not identify which direction the objective optical system rotating with the rotation of the insertion portion is oriented during insertion of the insertion portion, that is, the subject in which direction is being picked up. As configuration with the purpose of preventing such a state, an endoscope configured such that the distal end portion 7 provided at the distal end portion of the insertion portion 2 shown in Fig. 2 is configured as a distal end portion 7A in Fig. 6, for example, may be used.

The distal end portion 7A is configured by having a transparent member 201 formed by a transparent resin and the like which can transmit light as shown in Fig. 6. Inside the transparent member 201, an image pickup apparatus 301 that can pick up an image of a subject, convert the image of the subject to an image pickup signal and transmit the image pickup signal via radio, a liquid 302 such as water and the like on which the image pickup apparatus 301 can float, and a weight 303 connected to the image pickup apparatus 301 so that a field of view direction of the image pickup apparatus 301 maintains a predetermined diagonal direction to the insertion axis direction all the time and is formed by a member with a specific gravity larger than that of the liquid 302.

An exterior of the image pickup apparatus 301 is configured, as shown in Fig. 6, having an exterior member 311 with a section in the U-shape and a cover member 311 a substantially in a semispherical shape attached in a water-tight manner by an adhesive to an open end on the distal end side of the exterior member 311. The cover member 311 a is formed by a transparent resin and the like that can transmit light. Moreover, the image pickup apparatus 301 is hollow at least in a part of the inside. By means of the configuration of the image pickup apparatus 301 and the weight 303, the image pickup apparatus 301 can maintain a field of view in a predetermined diagonal direction all the time to the insertion axis direction while being floated on the liquid 302 all the time.

In a portion, which is an inside hollow portion in a capsule shape having the exterior member 311 and the cover member 311 a and corresponds to substantially the center of an arc of the semi-sphere of the cover member 311 a, an objective optical system 312 that forms an image of a subject incoming through the cover member 311a is arranged in a state contained in a frame body 313.

At the image forming position of the objective optical system 312, a CCD 314 is arranged. Also, an LED 315 emitting and radiating illumination light is arranged on the same plane around the frame body 313 containing the objective optical system 312.

In the inside hollow portion of the exterior member 311 on the rear end side of the CCD 314, an image pickup apparatus control portion 316 that controls creation of an image pickup signal photoelectrically converted by driving and controlling the CCD 314 and turned-on/-off of the LED 316, a communication processing portion 317 that brings the image pickup signal outputted from the image pickup apparatus control portion 316 into a state transmittable via radio, a transmission portion 318 that transmits an image pickup signal outputted from the communication processing portion 317 to the outside via radio, and a battery 319 that supplies power source used for driving of the image pickup apparatus control portion 316 and the communication processing portion 317.

The CCD 314, the LED 315, the image pickup apparatus control portion 316, the communication processing portion 317, and the transmission portion 318 are arranged on a substrate, not shown, respectively. Also, each substrate on which the CCD 314, the LED 315, the image pickup apparatus control portion 316, the communication processing portion 317, and the transmission portion 318 are arranged is connected by a flexible substrate, not shown.

A receiving portion 320 that receives an image pickup signal outputted from the transmission portion 318 via radio and then, outputs the image pickup signal to the video processor 5 through the signal line 320a and the like is arranged at a predetermined position (inside the insertion portion 2) outside the transparent member 201.

As mentioned above, the image pickup apparatus 301 of the distal end portion 7A can pick up an image of a subject present in a field of view in a predetermined diagonal direction all the time while being floated on the liquid 302 even if the insertion portion 2 is rotated. Thus, the user can easily identify in which direction an image of the subject is picked up from a body position of the examinee into which the insertion portion having the distal end portion 7A is inserted.

The above-mentioned image pickup apparatus 301 is not limited to one capable of transmission of a signal via radio but may be one with configuration that can achieve substantially the similar effect to the above-mentioned effect. For example, that may be configured as an image pickup apparatus 301A shown in Fig. 7 that transmits a signal by wire (through a signal line).

The image pickup apparatus 301A has configuration in which the communication processing portion 317, the transmission portion 318, and the battery 319 are removed from the image pickup apparatus 301 and a signal line 401a is connected to the image pickup apparatus control portion 316. And the image pickup signal outputted from the image pickup apparatus control portion 316 is outputted to signal line 401b through a connector portion 402a and a connector portion 402b provided at an end portion of the signal line 401a.

The connector portion 402a has the end portion side extending onto the outer surface of the connector portion 402a as shown in Fig. 8 and includes a connector 403a having a water-proof structure against the liquid 302 and a rubber packing 404a mounted on the end portion side of the connector 403a. A state of the connector portion 402a shown in Fig. 8 when seen from an arrow S direction is as shown in Fig. 9.

The connector portion 402b is, as shown in Fig. 8, arranged at a predetermined position (of the insertion portion 2) at the end portion of the signal line 401b and outside the transparent member 201 and has a connector 403b having a water-proof structure against the liquid 302.

And the connector portion 402a and the connector portion 402b shown in Fig. 8 are connected as in a state shown in Fig. 10. Specifically, the connector portion 402a and the connector portion 402b are connected in a state, as in Fig. 10, where the signal lines 401a and 401b are electrically conducted by bringing the connector 403b into contact with the connector 403a and a part of a contact portion 404b of the connector portion 402b is brought into contact with the rubber packing 404a. That is, the connector portion 402a and the connector portion 402b are connected in a state each of them is not fixed. Therefore, even if the insertion portion 2 is being rotated, the image pickup apparatus 301A can pick up an image of a subject present in a field of view in a predetermined diagonal direction all the time while being floated on the liquid 302.

It is needless to say that the present invention is not limited to each of the above-mentioned embodiments but capable of various changes and variations in a range not departing from the gist of the invention.

This application is filed while claiming priority of Japanese Patent Application No. 2006-256296 filed in Japan on September 21, 2006, and the above disclosed contents are incorporated in the present application description, claims and drawings by reference.

## Claims

1. An endoscope system comprising:
an elongated insertion portion;
an image pickup portion provided with an objective optical system having an optical axis diagonal to a longitudinal axis direction of the insertion portion and that picks up an image of a subject present in a field of view of the objective optical system and can rotate around the longitudinal axis of the insertion portion;
a rotation angle detection portion that detects a rotation angle of the image pickup portion;
an image pickup control portion that can have images of a plurality of subjects picked up while the image pickup portion makes one rotation by controlling the image pickup portion on the basis of the rotation angle detected by the rotation angle detection portion; and
a signal processing portion that carries out signal processing for having each image of the plurality of subjects displayed on one display portion.

2. The endoscope system according to claim 1, wherein the image pickup control portion has the image of the subject picked up each time the rotation angle of the image pickup portion reaches a plurality of predetermined angles.

3. The endoscope system according to claim 1, further comprising a rotation speed setting portion that can set a value of a rotation speed of the image pickup portion and a rotation speed control portion that controls the rotation speed of the image pickup portion to a value set by the rotation speed setting portion, wherein
the image pickup control portion sets a timing at which the image pickup portion picks up an image of the subject on the basis of the rotation speed set by the rotation speed setting portion.

4. The endoscope system according to claim 2, further comprising a rotation speed setting portion that can set a value of a rotation speed of the image pickup portion and a rotation speed control portion that controls the rotation speed of the image pickup portion to a value set by the rotation speed setting portion, wherein
the image pickup control portion sets a timing at which the image pickup portion picks up an image of the subject on the basis of the rotation speed set by the rotation speed setting portion.

5. The endoscope system according to claim 1, wherein each image of the plurality of subjects displayed on the one display portion is arranged according to a rotation direction of the image pickup portion.

6. The endoscope system according to claim 2, wherein each image of the plurality of subjects displayed on the one display portion is arranged according to a rotation direction of the image pickup portion.

7. The endoscope system according to claim 3, wherein each image of the plurality of subjects displayed on the one display portion is arranged according to a rotation direction of the image pickup portion.

8. The endoscope system according to claim 4, wherein each image of the plurality of subjects displayed on the one display portion is arranged according to a rotation direction of the image pickup portion.

9. The endoscope system according to claim 1, wherein each image of the plurality of subjects displayed on the one display portion is arranged according to a rotation angle of the image pickup portion.

10. The endoscope system according to claim 2, wherein each image of the plurality of subjects displayed on the one display portion is arranged according to a rotation angle of the image pickup portion.

11. The endoscope system according to claim 3, wherein each image of the plurality of subjects displayed on the one display portion is arranged according to a rotation angle of the image pickup portion.

12. The endoscope system according to claim 4, wherein each image of the plurality of subjects displayed on the one display portion is arranged according to a rotation angle of the image pickup portion.

13. The endoscope system according to claim 5, wherein each image of the plurality of subjects displayed on the one display portion is arranged according to a rotation angle of the image pickup portion.

14. The endoscope system according to claim 6, wherein each image of the plurality of subjects displayed on the one display portion is arranged according to a rotation angle of the image pickup portion.

15. The endoscope system according to claim 7, wherein each image of the plurality of subjects displayed on the one display portion is arranged according to a rotation angle of the image pickup portion.

16. The endoscope system according to claim 8, wherein each image of the plurality of subjects displayed on the one display portion is arranged according to a rotation angle of the image pickup portion.
